# EUROPEAN PATENT SPECIFICATION

(11) **EP 0 649 727 B1**
(45) Date of publication and mention of the grant of the patent: **15.05.2002**
(21) Application number: 94116668.8
(22) Date of filing: 21.10.1994
(51) Int. Cl.: B29C 65/20, A61M 39/00, B29C 65/78

(54) **Total containment welding of plastic tubes**
Schweissen von Kunststoffrohren unter hermetischen Abschluss
Soudage hermétique de tubes en matière plastique

(30) Priority: 22.10.1993 US 139833; 29.11.1993 US 158505; 15.08.1994 US 290548
(43) Date of publication of application: 26.04.1995
(73) Proprietor: DENCO, INC., Wilmington, Delaware 19899-2207 (US)
(72) Inventor: Ivansons, Ivars V., Newark, DE 19711 (US); Ivansons, Valdis, Wilmington, DE 19810 (US); Spencer, Dudley W.C., Wilmington, DE 19803 (US)
(74) Representative: Wagner, Karl H., Dipl.-Ing.

(56) References cited:
- EP-A- 0 208 004
- EP-A- 0 471 953
- EP-A- 0 483 478
- EP-A- 0 508 474
- EP-A- 0 583 582
- EP-A- 0 599 057
- US-A- 4 793 880

## Description

### Background of the Invention

The present invention is directed to the total containment welding of plastic tubes. Various prior art exists disclosing different approaches for welding plastic tubes together. Prior patents disclose processes where the weld connection depends on the melt rheology of plastic resulting in non-uniform size of the weld connection. No attempt was made in the prior art to gain control of the size of the weld connection.

Prior art also exists disclosing different approaches to disconnect and seal plastic tubes. Such prior art patents relate to processes where a single clamp jaw of a holder is used to shut off the tubing and to form the seal during disconnect resulting in uncontrolled, nonuniform sealing process.

Further, attention is drawn to EP-A-483 478 which relates to a device for total containment welding of plastic tubes. In said device, plastic tubes are welded by placing a pair of tubes in a pair of aligned holders, with each tube folded towards itself. A wafer is moveably mounted in the gap between the tubes and is heated to cause the ends of the tubes to melt without actually cutting through the tubes. The melted ends of the opposed tubes can be pressed into contact with each other to form a welded connection.

In accordance with the present invention, a device for selectively connecting and disconnecting plastic tubes and a wafer for use in a device for selectively connecting and disconnecting plastic tubes as set forth in claims 1 and 11, respectively, are provided.

Preferred embodiments of the invention are claimed in the dependent claims.

### summary of the Invention

An object of this invention is to provide techniques for the sterile or total containment welding of plastic tubes.

A further object of this invention is to provide such techniques which are particularly adaptable for use with the melt/wipe system.

A still further object of this invention is to provide an improved clamp jaw construction for effectively resulting in a sterile sealing of the plastic tubes.

In accordance with this invention a pair of aligned tube holders are spaced from each other to form a gap between the tube holders. Each of the tube holders has a first clamp jaw and a second clamp jaw movable relative to each other to selectively releasably clamp a plastic tube in a general plane between the first clamp jaw and second clamp jaw with a portion of the plastic tube extending into the gap. A wafer is mounted for movement through the gap. The wafer is in the form of a flat plate having opposite sides, each of which is disposed toward a respective set of first and second clamp jaws. Each of the sides of the wafer has an outwardly extending scoop generally located in the plane between the first and second clamp jaws for removing plastic material from the plastic tube as the wafer moves through the gap to control the size of the weld connection.

A further feature of the invention is the provision of a structural cam element on each holder spaced apart to create a precise spacing between the sets of clamp jaws for assuring the proper position of the wafer as it moves through the gap.

In accordance with a further feature of this invention each of the first and second clamp jaws is of double jaw construction having a base member or fixed jaw to which is mounted a spring loaded movable jaw. The spring loaded jaw is preferably mounted to the base member by means of a pressure plate remote from the clamping end of the jaw. The spring loaded jaw element effectively provides a sterile seal of its plastic tube.

### The Drawings:

Figure 1 is a top plan view of a device for selectively connecting and disconnecting plastic tubes;
Figure 2 is a front elevational view in section of the device shown in Figure 1;
Figure 3 is a rear elevational view in section of the device shown in Figures 1-2;
Figures 4A-4E are schematic showings of the use of the device shown in Figures 1-3 for the connect process;
Figures 5A-5E are schematic showings of the use of the device of Figures 1-3 for the disconnect process;
Figure 6 is a side elevational view of a wafer used in the device of Figures 1-5;
Figure 7 is a perspective view of the wafer shown in Figure 6;
Figure 8 is a perspective view of a clamp jaw used in the device of Figures 1-3;
Figure 9 is a side elevational view partially broken away of a sealed tube having a bag attached thereto wherein the tube is sealed by use of the device of Figures 1-3;
Figure 10 is a top plan view of the sealed tube shown in Figure 9;
Figure 11 is a cross-sectional view illustrating the seal removal and thickness control areas of a plastic tube in accordance with this invention;
Figure 12 is a graph showing compression distance to effect a high strength seal;
Figure 13 is a side elevational view of a modified form of wafer in accordance with this invention;
Figure 14 is a perspective view of a wafer shown in Figure 13;
Figure 15 is a cross-sectional view in elevation illustrating use of the wafer shown in Figures 13-14;
Figure 16 is a cross-sectional view in elevation of a butt welded tube using the wafer shown in Figures 13-15;
Figure 17 is a side elevational view of a further wafer in accordance with this invention;
Figures 17A and 17B are side elevational views of a portion of a wafer similar to that of Figure 17 showing alternate practices of this invention;
Figure 18 is a top plan view of the wafer shown in Figure 17;
Figures 19 and 20 are front and rear elevational views of the wafer shown in Figures 17-18;
Figure 21 is a bottom plan view of the wafer shown in Figures 17-20;
Figure 22 is a perspective view of the wafer shown in Figures 17-20;
Figure 23 is a side elevational view showing the wafer of Figures 17-22 mounted in a holder;
Figure 24 is a top plan view of the holder/wafer shown in Figure 23;
Figures 25 and 26 are front and rear elevational views of the wafer/holder shown in Figures 23-24;
Figure 27 is a bottom plan view of the wafer/holder shown in Figures 23-26;
Figure 28 is a perspective view of the wafer/holder shown in Figures 23-27;
Figure 29 is a top plan view of a portion of a total containment device in which the wafer of Figures 17-28 could be used; and
Figure 30 is a cross-sectional view taken through Figure 29 along the line 30-30.

### Detailed Description

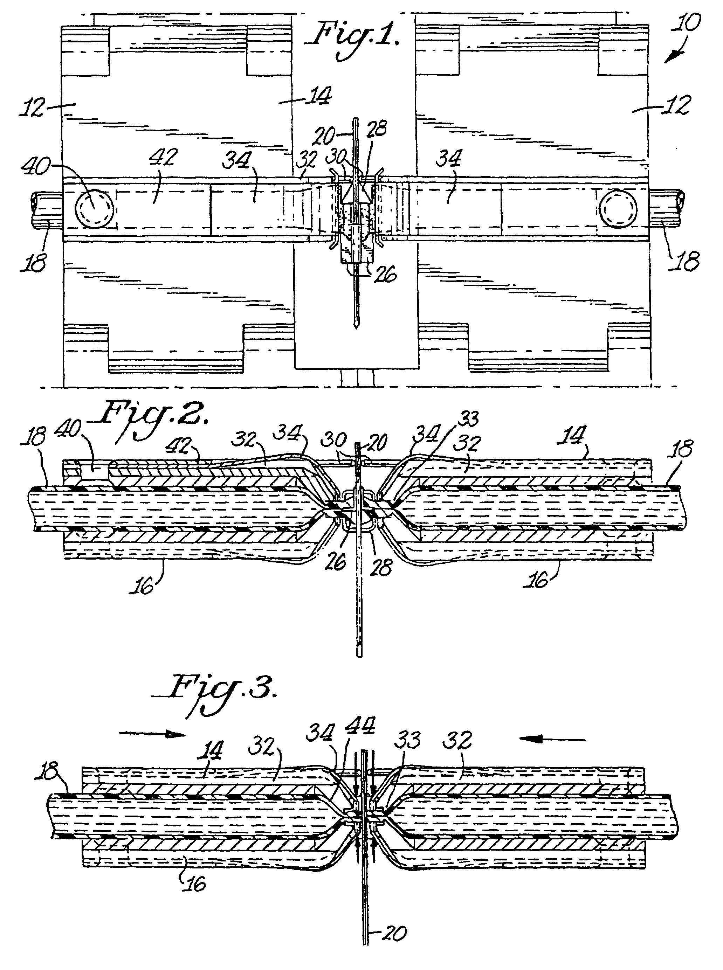

Figures 1-3 illustrate a welder 10 in accordance with this invention. In general, welder 10 is of the construction of the welder disclosed in U.S. Patent No. 5,279,685 the details of which are incorporated herein by reference thereto. Welder 10 includes a pair of aligned tube holders 12,12. Each tube holder includes an upper jaw 14 and a lower jaw 16 movable relative to each other to selectively releasably clamp a plastic tube 18 between each set of upper jaw and lower jaw. The tube holders are spaced apart to form a gap between the tube holders 12,12. The ends of the tubes 18,18 extend into the gap slightly spaced from each other. As later described a wafer 20 is moved through the gap and preferably is heated for melting the ends of the tubes so that the melted tube ends could be pressed into contact with each other to connect the tube ends for forming a single unitary tube. Preferably, this is accomplished by a melt/wipe technique as disclosed in the various parent applications.

The structure of the tube holders and clamp jaws is generally similar to that of U.S. Patent No. 5,279,685.

Figures 6-7 in particular show the details of wafer 20. As shown therein wafer 20 is in the form of a flat plate having a pair of opposite sides 22,22. Wafer 20 also includes a cut-out or notch 24 for engagement by a pawl in the home position of the wafer in the manner described in U.S. Patent No. 5,279,685. Wafer 20 also includes a pair of wings 26 as described in the various parent applications.

Wafer 20 is unique in that it also includes a scoop 28 on each of its sides. Scoop 28 is located generally in line with wings 26 which would be at an elevation which is in the general plane of the clamped tubes 18 where each upper jaw 14 and lower jaw 16 press against the tube ends to flatten the tube ends. Thus, scoops 28 are in a position to contact the melted tubes.

As best illustrated in Figures 1-2 each holder includes a cam element 30 in the form of a pin located at the upstream portion of the gap. Cam elements 30,30 are spaced apart a distance generally equal to the thickness of wafer 20 where its flat sides do not include the scoops or wings. Cam elements 30,30 thus assure the proper positioning of wafer 20 as wafer 20 begins to move through the gap between the holders 12,12. The scoops 28,28 in combination with the cam elements 30,30 are used to control the plastic volume of the weld. The tube ends are preferably melted by the wiping action of the wafer and pressed into contact with each other to form a precise weld connection. The scoops act as debris collectors and make it possible to achieve a means of controlling the tube exterior flange sides for optimal strength and aesthetics. In this manner reliance on rheology of plastic to move excess away from the weld site is avoided. Figure 10, for example, illustrates a tube end where excess plastic has been removed by the scoops 28,28.

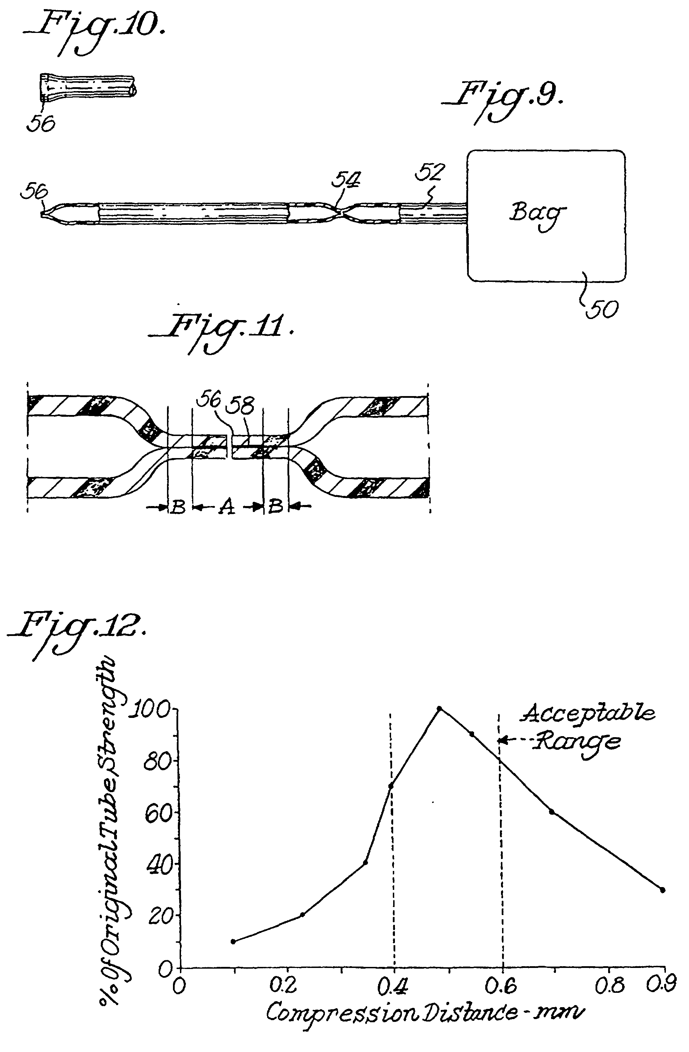

Figures 1-2 and 8 illustrate a further feature of this invention wherein each jaw member is of double jaw construction. As shown therein each jaw member includes a base portion or fixed jaw 32 which may be channel shaped to accommodate a spring arm 34. Spring arm 34 functions as a movable jaw. A pressure plate 36 is also disposed in the channel formed at the upper portion of base member 32 to press against spring arm 34. As illustrated in Figure 8 an elongated opening 38 extends through the base member 32, the spring arm 34 and the pressure plate 36. A suitable fastener, such as a rivet or bolt 40 (Figures 1-2) secures these members together. As a result, pressure plate 36 maintains spring arm 34 anchored over about half of its length with the other half hinged at the edge 42 of pressure plate 36.

The formation of a fixed base or jaw member 32 and the spring arm 34 thus results in a double jaw clamp jaw which may be considered spring loaded to effectively press against and seal the tube ends when the first and second clamp jaws 14,16 are moved relatively toward each other. With this double jaw technique a reliable strong tube seal 33 results. One pair of fixed jaws functions to shut off the tubing and preventing the tubing from slipping. The other pair of movable jaws form the seal 33 during the plastic melt process.

In a preferred practice of the invention the double jaw formation has the following characteristics. The fixed jaw spacing is 1.6 millimeters, the jaw tooth size is 0.3 millimeters, the width (flat tube) is 7.3 millimeters, the movable jaw force is 2 lbs., the length of the spring spacer or pressure plate is 16 millimeters.

Very strong seals are made by using wings that are long enough to create a smooth molten plastic pool inside the tubing. This is accomplished by locating the tooth 44 of the fixed jaw 32 as close as possible (e.g. 0.25 mm) to the front 46 of the jaw and by using the movable jaws. Each wing 26 must protrude beyond the tooth 44 of the fixed jaw and into the open inside the tubing.

The spring loaded double jaws insure tube seal as the wings 26 are removed. The spring loaded jaws at a 45° angle of end 46 provide guide edges that funnel the wafer 20 into the centered position. The fixed clamp jaws 32 clamp down to seal the liquid area and thus prevent escape of liquid. A final seal width which in the preferred range is 0.4 - 0.6 mm as shown in Figure 12 is preferable.

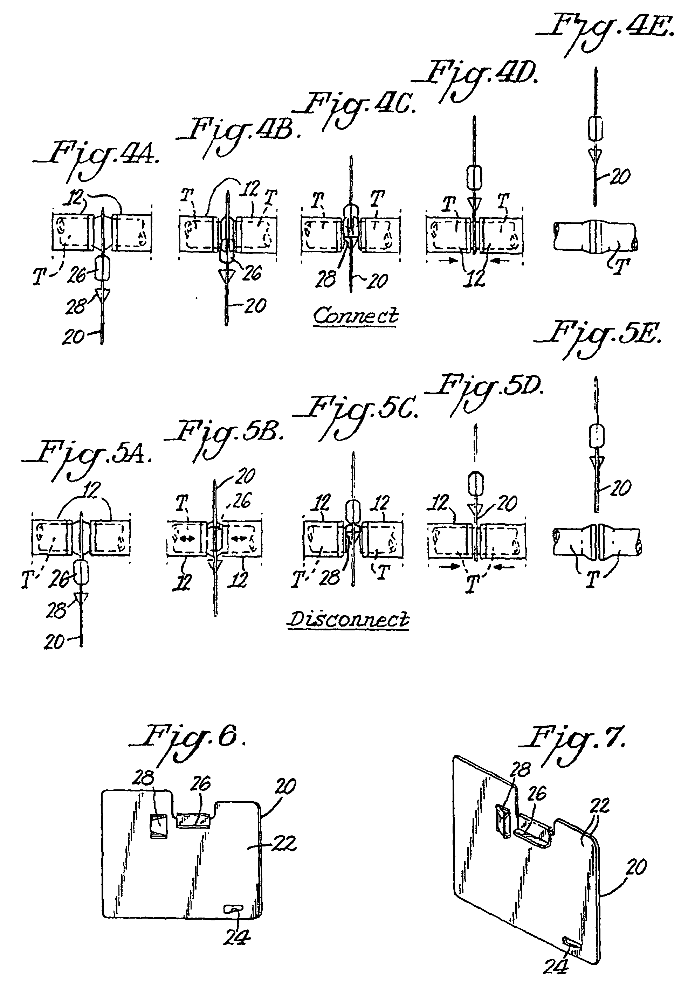

Figures 4A-4E illustrate the use of welder 10 in the connect process. As shown in Figure 4A the wafer 20 begins to enter the gap between the tube ends. The heated wafer preferably contacts or simply could be near the tube ends to melt the tube ends in the melt/wipe technique described in the various parent applications. As shown in Figure 4A the wings 26 and scoops 28 have not yet entered the gap. Although not illustrated in Figures 4A-4E the cam elements 30 would assure the proper positioning of the wafer 20 as it passes through the gap.

Figure 4B illustrates the next sequence in operation wherein the wings 26 begin to enter the inside of each flattened tube end.

Figure 4C shows the next sequence where the wings are moving out of the gap and scoops 28,28 are removing excess plastic material.

Figure 4D illustrates the next sequence of operation wherein the wafer is moving out of the gap at which time or slightly thereafter the holders 12,12 begin to move toward each other to press the heated ends of the tubes into contact with each other and thus result in the unitary tube T shown in Figure 4E.

Figure 5A-5E illustrates the same welder 10 used in a disconnect procedure. As shown therein a single tube T would be placed across the holders 12,12 spanning the gap between the holders. The heated wafer 20 would begin to cut through the tube in a melting operation as shown in Figure 5A.

Figure 5B illustrates the next sequence in operation wherein the wings 26 enter the cut tube portions.

Figure 5C illustrates the next sequence in operation wherein the scoops 28 remove excess plastic material.

Figure 5D illustrates the next sequence where the wafer is exiting from the gap. In this sequence the tube holders 12,12 may be moved toward the wafer to clean the sealed ends by a heating operation.

Figure 5E illustrates the two separated tube sections resulting from the procedure.

Figure 9 illustrates the use of the invention for connection or disconnection of a tube section to the tube section communicating with a receptacle or bag 50. As shown therein a bag 50 would have a tube section 52 permanently connected thereto. The arrangement of Figure 9 illustrates an unopen connection 54 of another tube section terminating in an end seal 56. The end seal may be, for example, 1.5 mm.

Figure 10 illustrates the flattened end seal 56 is illustrated in its plan view.

The invention may be used for connecting and/or disconnecting plastic tube sections to permit the replacement of various types of receptacles in a system. For example, U.S. Patent No. 5,141,592, the details of which are incorporated herein by reference thereto, describes various applications which may be utilized for practicing the present invention. In such procedures, periodically it would be desired to replace the bag or receptacle 50 with a different bag. For example, the receptacle or bag 50 could be a CAPD bag or a urinary drainage bag and it would be periodically desirable to replace the used bag with a new bag. Additionally, the bag or receptacle 50 may also advantageously be a blood processing receptacle.

The importance of gaining control over the distance, or compression of the molten plastic when making the weld can be understood by a simple analogy. For example, when joining the ends of waxed candles, to make a long candle from short ends, one finds very quickly that not pushing the ends close enough together makes a poor joint. There is not enough molten wax available to form a strong bond. Similarly, if one pushes too hard the molten wax is pushed out of the joint area to where the butt ends are pushing against cold wax. This also makes a weak joint. One learns quickly, therefore, there is an optimum amount of pushing that results in an acceptable joint.

In a general way, this analogy carries over to making strong sterile joints in plastic tubing. Unlike wax candles sterile connection joints require very reliable consistent highest strength welds. To achieve these conditions control of the plastic temperature and compression distance is extremely important. For example, the inventors have established the data shown in Figure 12 when using medical gray (class 6) PVC tubing 5.4 mm O.D. and 4.0 mm I.D. while maintaining a wafer temperature of 350°C.

As can be seen from Figure 12 the range for acceptable strength is extremely narrow and therefore the cam control of clamp distance as provided by the present invention is a novel and effective means of administering the controls.

Similarly, the use of cams located on the clamped surfaces can be used to control another vital area of making strong sterile connectings in tubing. When welds are made in tubes that contain liquid (as illustrated in Figures 2-3) it is necessary to clamp the tubes in the weld area to avoid liquid escape. During the welding process a membrane is formed within the tube that must be broken to reestablish the liquid flow in the lumen. If the membrane is too thin then liquid flow is not stopped. If the membrane is too thick the liquid flow can not be reestablished. Control of the membrane thickness therefore is important. This control is accomplished by controlling the position of the scoops 28. Figure 11 illustrates the control area B,B with the seal area A. The seal 58 takes place at portion 56. The scoops 28 under control of the cams 30 for accurate positioning removes the seal area A and the scoops are positioned to obtain reproducible and reliable thickness of the membrane. The invention has the advantages of maximizing the seal strength by controlling the compression distance. Additionally, the cams 30,30 engage the wafer 20 to control the penetration of the wings 26 during the disconnect procedure. The flange height is controlled by removing excess material with scoops 28,28. Cams 30,30 insure that the wafer 20 is properly positioned in a vertical orientation.

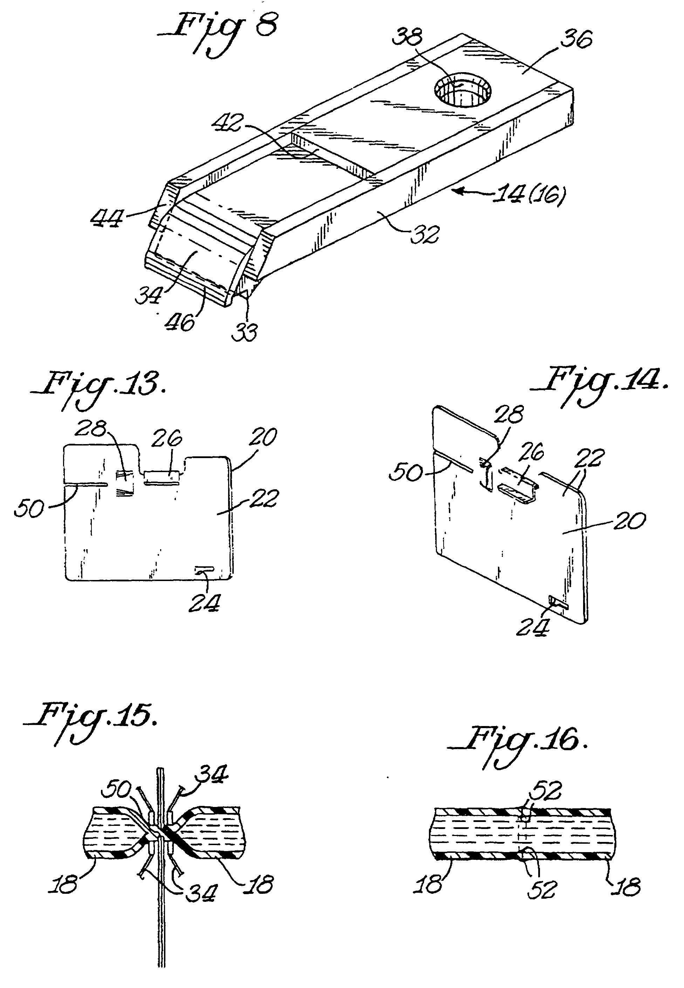

Figures 13-15 illustrate a modified form of wafer in accordance with this invention. The wafer shown therein would be of generally the same construction as wafer 20 shown in Figures 6-7. In Figures 13-15, however, the wafer also includes a slit 50 extending completely through the wafer downstream from scoop 28. As illustrated slit 50 extends inwardly from the downstream generally vertical trailing edge in a straight line generally horizontal direction toward the upstream generally vertical leading edge of wafer 20. Slit 50 terminates downstream from scoops 28. Slit 50 may be of any suitable dimension and preferably has a length of 7mm and a width of 0.5 mm. Slit 50 is disposed in line with scoop 28 and wing 26 on each of the sides 22 of wafer 20.

The advantage of providing a slit, such as slit 50 in the heated wafer downstream from the scoops 28 is to permit the molten material from the tubes 18 to begin contacting each other at an earlier time while the tubes are still in the area of the heated wafer. Figure 15, for example, shows the molten ends of tubes 18 to enter the slit 50 before the wafer 20 has completely cleared contact with the tubes 18,18. The resultant joint, which is illustrated in Figure 16, after the tubes have been opened into communication with each other is of increased strength leaving only a slight residue 52,52 at the joint.

The invention has the advantages of assured sterility and maximum weld strength. The invention could be used with a wide range of plastic materials such as PVC, polyurethane, hytrel, nylon, polyethylene, polypropylene and various compositions of Teflon. The invention results in improved aesthetics and in the control of the lumen re-opening requirements.

The present invention also includes features in the wafer to assure single use of a wafer. Advantageously, the preferred practice of this invention requires minimal modifications to the wafer and total containment device.

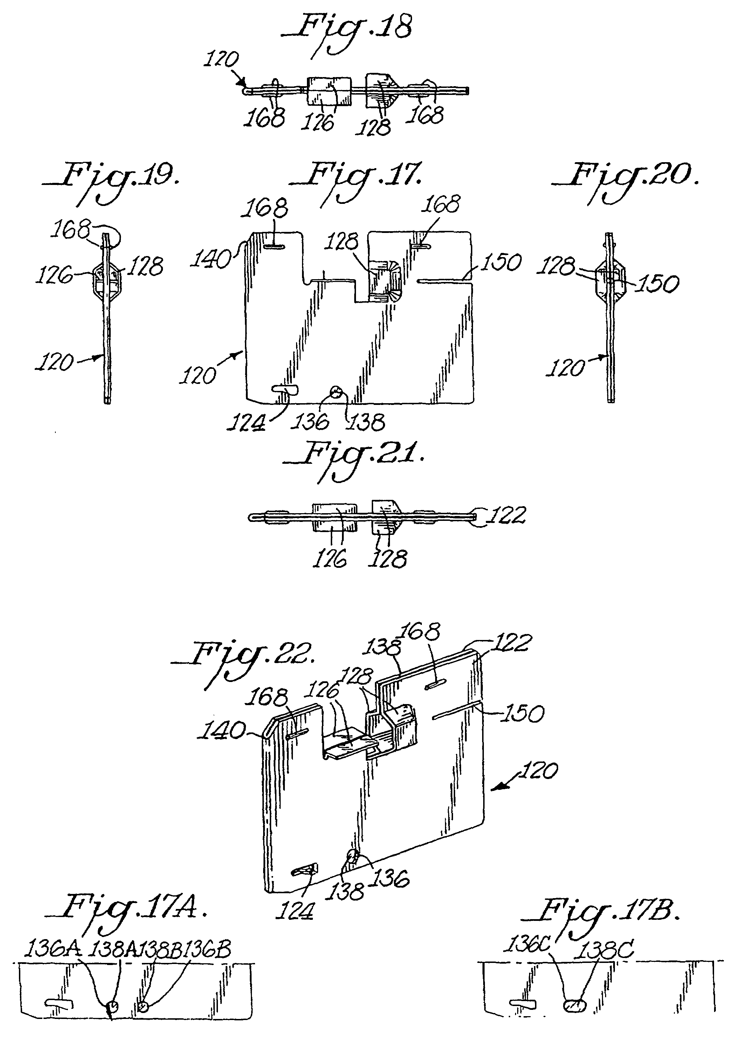

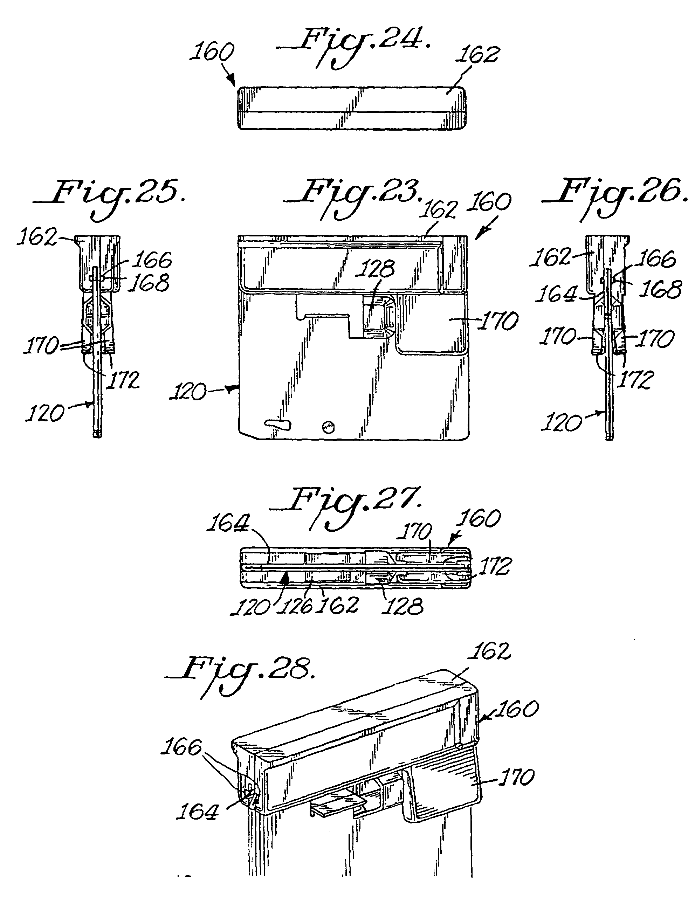

Figures 17-22 show the details of a single use wafer 120. As shown therein wafer 120 is in the form of a flat plate having a pair of opposite sides 122,122. Wafer 120 also includes a cut-out or notch 124 for engagement by a pawl in the home position of the wafer in the manner previously described with respect to wafer 120. Wafer 120 also includes a pair of wings 126.

Wafer 120 also includes a scoop 128 on each of its sides. Scoop 128 is located generally in line with wings 126 which would be at an elevation which is in the general plane of the clamped tubes 118 where each upper jaw and lower jaw press against the tube ends to flatten the tube ends. See Figure 29 and U.S. Patent No. 5,279,685. Thus, scoops 128 are in a position to contact the melted tubes.

The wafer 120 also includes a slit 150 extending completely through the wafer downstream from scoops 128. As illustrated slit 150 extends inwardly from the downstream generally vertical trailing edge in a straight line generally horizontal direction toward the upstream generally vertical leading edge of wafer 120. Slit 150 terminates downstream from scoops 128. Slit 150 may be of any suitable dimension and preferably has a length of 7mm and a width of 0.5 mm. Slit 150 is disposed in line with scoop 128 and wing 126 on each of the sides 122 of wafer 120.

In order to assure the single use of the wafer, wafer 120 is provided with a hole or aperture 136 at any suitable location such as being generally in line with notch 124. Aperture 136 is intended to be covered by a sensing material 138. This can be conveniently accomplished by forming the wafer as a plate which is folded upon itself at one end 140 so that the wafer 120 is of double thickness, as best shown in Figures 18 and 20-22. Prior to the complete folding the sensing material 138 is positioned to cover hole 136. Material 138 is preferably selected to be of material which would melt or otherwise be destroyed upon the heating of the wafer. Thus, once there has been a single use of the wafer, material 138 no longer covers aperture 136 and can not be sensed in an attempted second use of the wafer.

Figures 29-30 illustrate, in general, portions of the total containment device 110 of the type described in U.S. Patent No. 5,279,685. As shown therein device 110 includes a carriage 112 for moving the wafer through the device. U.S. Patent No. 5,279,685 discloses the inclusion of various sensors, such as for indicating that the wafer is properly loaded in the carriage and for detecting that the wafer is properly conditioned with respect to the clenching block. Figure 30 illustrates one such sensor 114 which, in the device of the parent patent, functions as the aperture blockage sensor. Sensor 114 is mounted in the path of movement of aperture 136 and thus would sense aperture 136 passing through the path of view of sensor 114. Thus, the present invention advantageously makes use of a sensor already in device 110 for its single use sensing operation. If desired, however, one of the other sensors or a completely additional sensor may be used for this purpose.

In operation, as wafer 120 is moved in a downstream direction sensor 114 views aperture 136. If there is sensing material 138 located in aperture 136 the material will be sensed. Such sensing could be in terms, for example, of a voltage reading which would differ from the voltage reading of the copper wafer material 120 and would differ from the voltage reading of a completely open hole 136. If the sensor 114 does not detect the proper voltage which would result from the proper sensing material 138 being in aperture 136 then the device 110 would be inactivated. This would mean that either the wafer 120 had been previously used and no longer has sensing material 138 or that there is a manufacturing defect and the sensing material was not mounted over hole 136.

When the sensing material 138 is properly detected and other device requirements are met, the wafer 120 is heated for use in the selective connect/disconnect of the plastic tubes 118,118. The heating operation results in a melting of sensing material 138 so that hole 136 is completely exposed.

The concept of sensing a condition of the wafer which would indicate whether or not the wafer has been previously used can be practiced in various manners. Figure 17A, for example, illustrates the provision of two holes 136A,136B, each with a sensing material 138A,138B. Sensing materials 138A and 138B may be the same or different materials. This arrangement would provide a more sophisticated reading to require a binary or sequential voltage reading from sensor 114 in order to prevent device 110 from being inactivated. Such more sophisticated reading would be desirable to make it more difficult for a user to attempt to insert sensing material back into wafer 120 after the wafer has already been used.

Cover material 138 is preferably a two mil thick polyethylene sheet which may contain carbon black. Such material could also be used as material 138A. Material 138B could likewise be the same material or could be a material having a different voltage characteristic.

Figure 17B shows yet another practice of this invention wherein the aperture 136C is an elongated slot. The cover material 138C could be the same as cover material 138 or could simply be any suitable material, preferably a transparent material having some indicia thereon which would be detected by the sensor. For example, the indicia could be a bar code or some form of alphanumeric code. The indicia could be visible to the naked eye or could be invisible to the naked eye since it is only necessary that the indicia be visible to the sensor.

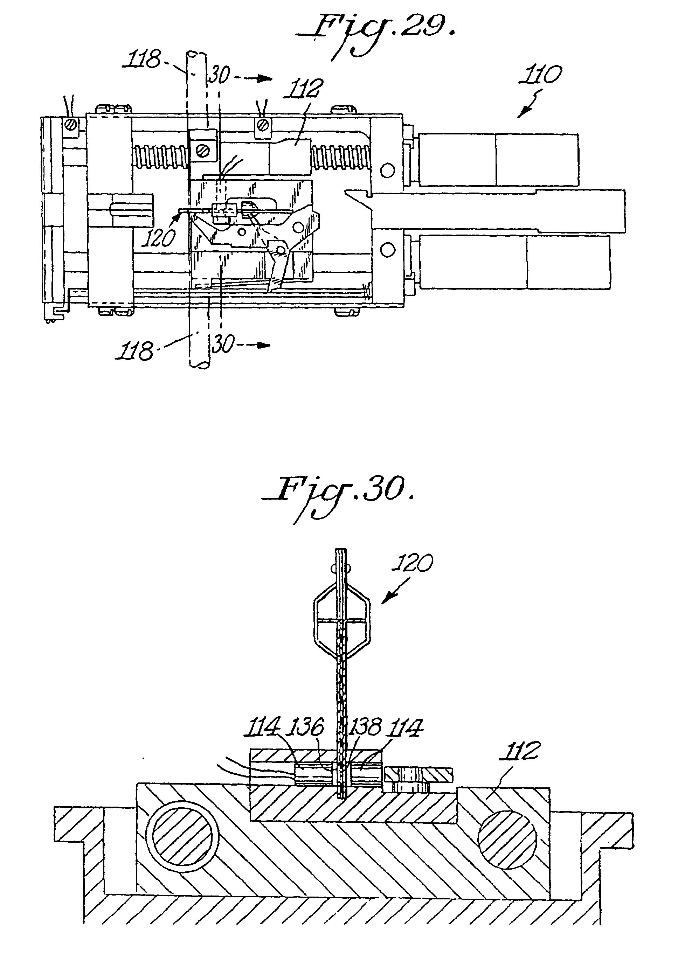

Figures 23-28 illustrate wafer 120 mounted in a suitable holder 160 which could be of the same general construction as the holder illustrated in Figure 14 of U.S. Patent No. 5,279,685. The purpose of the holder is to mount wafer in the carriage, during the initial movement of wafer 120. Holder 116 generally includes a split body 162 having a slot 164 into which wafer 120 would be slidably mounted. Interior of body 162 would include two registration recesses for receiving lugs 168 on wafer 120. Holder body 162 includes downward extensions 170 which have half round filets 172 to increase the frictional contact on wafer 120 by the spring action of the body extension 170 which thus act as spring arms.

In operation, wafer 120 would be mounted in holder 160 during the initial positioning of the wafer in carriage 112. As the wafer is moved downstream it is detached from holder 160 thereby exposing slit 150 which is initially covered by extension 170 of holder 160.

It is to be understood that the holder 160 illustrated in Figures 23-28 may be used with the type of wafer such as wafer 20 and is not limited to the type of wafer intended for single use purpose. Even where holder 160 is used for a single use wafer, the holder itself may be reused multiple times.

It should.be noted that the objects and advantages of the invention may be attained by means of any compatible combination(s) particularly pointed out in the appended claims.

## Claims

1. A device (10) for selectively connecting and disconnecting plastic tubes (18; T) comprising a pair of aligned tube holders (12, 12) spaced from each other to form a gap therebetween, each of said holders having a first clamp jaw (14) and a second clamp jaw (16) movable relative to each other to selectively releasably clamp a plastic tube (18) in a general plane between said first clamp jaw (14) and said second clamp jaw (16) with a portion of the plastic tube (18) extending into said gap, a wafer (20; 120) mounted for movement through said gap, said wafer (20; 120) being in the form of a flat plate having opposite sides (22, 22), each of said sides of said wafer (20; 120) being disposed toward a respective set of said first and second clamp jaws (14, 16), and each of said sides of said wafer having an outwardly extending scoop (28; 128) having an open end and otherwise being closed to form a collecting pocket, which is generally located in said plane between said first and second clamp jaws (14, 16) for removing plastic material from the plastic tubes (18) as said wafer (20, 120) moves through said gap.

2. The device (10) of claim 1 wherein each of said holders (12, 12) includes a cam (30) extending into said gap and being aligned with each other and spaced apart a distance generally equal to the thickness of said wafer (20; 120), and said cams (30) being located in the path of motion of said wafer (20; 120) at a portion of said wafer (20, 120) other than said scoops (28; 128).

3. The device (10) of claim 2 wherein each of said cams (30) comprises a pin mounted to its said holder (12) at a downstream portion of said gap.

4. The device (10) of any one of the preceding claims, wherein said wafer includes a wing (26; 126) on each of said sides, and each of said wings being generally in line with its respective scoop (28; 128) on the same side of respective wafer (20; 120).

5. The device (10) of claim 4 wherein said wafer is a heated wafer, and said open end of said scoops (28; 128) is disposed toward said wing (26; 126).

6. The device of any one of the preceding claims, wherein each of said jaws (14, 16) is in the form of a double jaw comprising a fixed jaw (32) and a movable jaw (34), said fixed jaw (32) being a base portion (32), and said movable jaw (34) being a spring arm (34) mounted to said base portion (32).

7. The device (10) of claim 6 wherein said base portion (32) includes a channel, said spring arm (34) being mounted in said channel, a pressure plate (36) being mounted in said channel, against said spring arm (34), and a fastener (40) securing said pressure plate (36) and said spring arm (34) to said base portion (32) in said channel.

8. The device of any one of the preceding claims wherein said wafer (20; 120) includes a generally straight line slit (50; 150) extending from an edge of said wafer (20; 120) completely therethrough, and said slit (50; 150) being generally in line with and downstream from said scoops (28; 128).

9. The device of claim 1, wherein each of said holders (12, 12) has a cam (30) extending into said gap, and said cams (30) being aligned with each other and spaced apart a distance generally equal to the thickness of said wafer (20; 120) between said sides of said wafer for guiding said wafer as said wafer moves through said gap.

10. The device (10) of claim 9, wherein each of said cams (30) comprises a pin mounted to said holder (12) at the downstream portion of said gap.

11. A wafer (20; 120) for use in a device (10) for selectively connecting and disconnecting plastic tubes (18; T), said wafer being in the form of a heated flat plate having opposite sides (22, 22), each of said sides having an outwardly extending scoop (28; 128), each of said scoops having an open end and otherwise being closed to form a collecting pocket, and each of said scoops (28; 128) being located at the same portion of its respective side (22) as the other of said scoops whereby said scoops (28; 128) form mirror images of each other.

12. The wafer of claim 11, including a wing (26; 126) extending outwardly from each of said sides (22) spaced from and generally in line with a respective one of said scoops (28; 128).

13. The wafer of claim 12, wherein said scoops (28; 128) and said wings (26; 126) are located at the upper end of each of said sides (22), and a notch (24; 124) being formed in the lower end of said wafer (20; 120).

14. The wafer of any one of claims 11 to 13, including a generally straight line slit (50; 150) extending from an edge of said plate completely through said plate, and said slit being generally in line with and downstream from said scoops (28, 128).

15. The wafer of any one of claims 11 to 14, including an aperture (136) extending completely through said plate, and a sensing material (138) covering said aperture (136).

## Patentansprüche

1. Vorrichtung (10) zum selektiven Verbinden und Trennen von Plastikschläuchen (18; T), wobei folgendes vorgesehen ist:
ein Paar von ausgerichteten Schlauchhaltern (12, 12) voneinander beabstandet zur Bildung eines Spaltes dazwischen, wobei jeder der erwähnten Halter einen ersten Klemmgreifer (14) und einen zweiten Klemmgreifer (16) aufweist, und zwar relativ zueinander beweglich, um selektiv einen Plastikschlauch (18) lösbar festzuklemmen, und zwar in einer allgemeinen Ebene zwischen dem ersten Klemmgreifer (14) und dem zweiten Klemmgreifer (16), wobei ein Teil des Plastikschlauches (18) sich in den erwähnten Spalt erstreckt,
eine Scheibe (20; 120) angebracht zur Bewegung durch den Spalt, wobei die Scheibe (20, 120) in der Form einer flachen Platte ausgebildet ist, die entgegengesetzt liegende Seiten (22, 22) aufweist, wobei jede der erwähnten Seiten der erwähnten Scheibe (20, 120) zu einem entsprechenden Satz der ersten und zweiten Klemmglieder (14, 16) hinweisend angeordnet ist, und
wobei jede der erwähnten Seiten der Scheibe eine sich nach außen erstrekkende Ausbuchtung bzw. Wölbung (28; 128) aufweist mit einem offenen Ende und ansonsten geschlossen, um eine Sammeltasche zu bilden, die im allgemeinen in der erwähnten Ebene angeordnet ist zwischen den ersten und zweiten Klemmgreifern (14, 16) zur Entfernung von Kunststoffmaterial von den Plastik- oder Kunststoffschläuchen (18), wenn die erwähnte Scheibe (20, 120) durch den Spalt sich bewegt.

2. Die Vorrichtung (10) nach Anspruch 1, wobei jeder der Halter (12, 12) einen Nocken (30) aufweist, der sich in den Spalt erstreckt und die miteinander ausgerichtet sind und um einen Abstand beabstandet sind im allgemeinen gleich der Dicke der Scheibe (20, 120), und wobei die Nocken (30) in der Bewegungsbahn der Scheibe (20, 120) angeordnet sind, und zwar an einem Teil der Scheibe (20, 120), der sich von den Ausbuchtungen (28; 128) unterscheidet.

3. Die Vorrichtung (10), wobei die Nocken (30) einen Stift aufweisen, der an seinem erwähnten Halter (12) an einem stromabwärts gelegenen Teil des Spaltes angebracht ist.

4. Die Vorrichtung (10) nach einem der vorhergehenden Ansprüche, wobei die Scheibe einen Flügel (26; 126) auf jeder der erwähnten Seiten aufweist, und wobei jeder der erwähnten Flügel im allgemeinen in Linie mit seiner entsprechenden Ausbuchtung (28; 128) liegt, und zwar auf der gleichen Seite der entsprechenden Scheibe (20, 120).

5. Die Vorrichtung (10) nach Anspruch 4, wobei die Scheibe eine beheizte Scheibe ist und wobei das erwähnte offene Ende der erwähnten Ausbuchtungen (28, 128) zu dem erwähnten Flügel (26; 126) hin angeordnet ist.

6. Die Vorrichtung nach einem der vorhergehenden Ansprüche, wobei jeder der erwähnten Klemmgreifer (14, 16) in der Form eines Doppelklemmgreifers ist, und zwar einen festen Greifer oder Klaue (32) aufweisend und einen beweglichen Greifer oder Klaue (34) aufweisend, wobei der feste Greifer (32) einen Basisteil (32) besitzt und wobei der bewegliche Greifer (34) ein Federarm (34) ist, und zwar angebracht an dem erwähnten Basisteil (32).

7. Die Vorrichtung (10) nach Anspruch 6, wobei der erwähnte Basisteil (32) einen Kanal aufweist, wobei der Federarm (34) in dem erwähnten Kanal angebracht ist und wobei ferner eine Druckplatte (36) in dem Kanal angebracht ist, und zwar entgegen dem Federarm (34), und wobei eine Befestigungsvorrichtung (40) die erwähnte Druckplatte (36) und den Federarm (34) an dem Basisteil (32) in dem Kanal befestigt.

8. Die Vorrichtung nach einem der vorhergehenden Ansprüche, wobei die Scheibe oder Wafer (20, 120) einen im allgemeinen geradlinigen Schlitz (50, 150) aufweist, und der sich von einer Kante der Scheibe (20, 120) vollständig da hindurch erstreckt, und wobei der erwähnte Schlitz (50; 150) im allgemeinen auf einer Linie liegt mit den Ausbuchtungen (28, 128) und stromabwärts von diesen.

9. Die Vorrichtung nach Anspruch 1, wobei jeder der erwähnten Halter (12, 12) einen Nocken (30) aufweist, der sich in den Spalt erstreckt, und wobei die Nocken (30) miteinander ausgerichtet sind und voneinander um einen Abstand im allgemeinen gleich der Dicke der Scheibe (20, 120) beabstandet sind, und zwar zwischen den erwähnten Seiten der Scheibe zum Führen der Scheibe, wenn sich die Scheibe durch den Spalt bewegt.

10. Die Vorrichtung (10) nach Anspruch 9, wobei jeder der erwähnten Nokken (30) einen Stift aufweist, und zwar angebracht an dem Halter (12) an einem stromabwärts gelegenen Teil des Spalts.

11. Ein Wafer (20; 120) zur Verwendung in einer Vorrichtung (10) zum selektiven Verbinden und Trennen von Plastik- oder Kunststoffschläuchen (18; T), wobei die Scheibe in der Form einer beheizten flachen Platte mit entgegengesetzt liegenden Seiten (22, 22) vorgesehen ist, wobei jede der erwähnten Seiten eine sich nach außen erstreckende Erweiterung bzw. Ausbuchtung (28, 128) aufweist, wobei jede der erwähnten Ausbuchtungen ein offenes Ende besitzt und ansonsten geschlossen ist, um eine Sammeltasche zu bilden, und wobei ferner jede der Ausbuchtungen (28; 128) an dem gleichen Teil zu einer entsprechenden Seite (22) angeordnet ist, wie die andere der erwähnten Ausbuchtungen, wodurch die Ausbuchtungen (28; 128) Spiegelbilder voneinander bilden.

12. Die Scheibe nach Anspruch 11 mit einem Flügel (26; 126), der sich nach außen von jeder der erwähnten Seiten (22) erstreckt, und zwar beabstandet von und im allgemeinen auf einer Linie mit einer entsprechenden der Ausbuchtungen (28; 128).

13. Die Scheibe nach Anspruch 12, wobei die erwähnten Ausbuchtungen (28; 128) und die Flügel (26; 126) am oberen Ende jeder der erwähnten Seiten (22) angeordnet sind, und wobei eine Kerbe (24, 124) in dem unteren Ende der erwähnten Scheibe (20; 120) ausgebildet ist.

14. Die Scheibe nach einem der Ansprüche 11 bis 13 mit einem im allgemeinen geradlinigen Schlitz (50; 150), und zwar sich erstreckend von einer Kante der erwähnten Platte vollständig durch die erwähnte Platte, und wobei der Schlitz im allgemeinen auf einer Linie mit und stromabwärts gelegen von den Ausbuchtungen (28; 128) liegt.

15. Die Scheibe nach einem der Ansprüche 11 bis 14 einschließlich einer Öffnung (136), die sich vollständig durch die Platte erstreckt, und wobei ein Abfüllmaterial (138) die erwähnte Öffnung bzw. Apertur (136) abdeckt.

## Revendications

1. Dispositif (10) pour connecter et déconnecter de manière sélective des tubes en matière plastique (18 ; T) comprenant un couple de supports de tube alignés (12, 12) disposés à distance l'un de l'autre de manière à former un intervalle entre eux, chacun des supports comprenant une première mâchoire de pince (14) et une seconde mâchoire de pince (16) mobiles l'une par rapport à l'autre pour pincer de manière sélective et amovible un tube en matière plastique (18) sensiblement selon un plan entre la première mâchoire de pince (14) et la seconde mâchoire de pince (16) avec une partie du tube en matière plastique (18) s'étendant dans ledit intervalle, une plaque (20 ; 120) montée pour se déplacer à travers ledit intervalle, la plaque (20 ; 120) ayant la forme d'une plaque plate ayant des cotés opposés (22, 22), chacun des cotés de la plaque (20 ; 120) étant disposé en regard d'un ensemble respectif de première et seconde mâchoires de pince (14, 16) et chacun des cotés de la plaque comprenant un godet s'étendant vers l'extérieur (28 ; 128) comprenant une extrémité ouverte et étant par ailleurs fermé de manière à former une poche de ramassage, qui est sensiblement situé dans le plan entre les première et seconde mâchoires de pince (14, 16) pour retirer de la matière plastique des tubes en matière plastique (18) lorsque la plaque (20 ; 120) se déplace à travers ledit intervalle.

2. Dispositif (10) selon la revendication 1, dans lequel chacun des supports (12, 12) comprend une came (30) s'étendant dans ledit intervalle, les cames étant alignées l'une par rapport à l'autre et séparées d'une distance sensiblement égale à l'épaisseur de la plaque (20 ; 120), et les cames (30) étant situées sur le chemin de déplacement de la plaque (20 ; 120) au niveau d'une partie de la plaque (20, 120) autre que celle comprenant les godets (28 ; 128).

3. Dispositif (10) selon la revendication 2, dans lequel chacune des cames (30) comprend une tige montée sur son support (12) au niveau d'une partie aval dudit intervalle.

4. Dispositif (10) selon l'une quelconque des revendications précédentes, dans lequel la plaque comprend une aile (26 ; 126) sur chacun de ses cotés, et dans laquelle chacune des ailes est sensiblement alignée avec le godet (28 ; 128) situé sur le même coté de la plaque (20 ; 120).

5. Dispositif (10) selon la revendication 4, dans lequel la plaque est une plaque chauffée, l'extrémité ouverte des godets (28, 128) étant disposée vers les ailes (26 ; 126).

6. Dispositif selon l'une quelconque des revendications précédentes, dans lequel chacune des mâchoires (14, 16) a la forme d'une double mâchoire comprenant une mâchoire fixe (32) et une mâchoire mobile (34), la mâchoire fixe (32) étant une partie de base (32) et la mâchoire mobile (34) étant un bras de ressort (34) monté sur la partie de base (32).

7. Dispositif (10) selon la revendication 6, dans lequel la partie de base (32) comprend une gorge, le bras de ressort (34) étant monté dans la gorge, une plaque de pression (36) étant montée dans la gorge contre le bras de ressort (34) et un dispositif d'assemblage (40) fixant la plaque de pression (36) et le bras de ressort (34) à la partie de base (32) dans ladite gorge.

8. Dispositif selon l'une quelconque des revendications précédentes dans lequel la plaque (20 ; 120) comprend une fente sensiblement rectiligne (50 ; 150) s'étendant à partir d'un bord de la plaque (20 ; 120) et la traversant complètement, ladite fente (50 ; 150) étant sensiblement dans l'alignement et en aval des godets (28 ; 128).

9. Dispositif selon la revendication 1, dans lequel chacun des supports (12, 12) comprend une came (30) s'étendant dans ledit intervalle, et dans lequel les cames (30) sont alignées l'une par rapport à l'autre et séparées d'une distance sensiblement égale à l'épaisseur de la plaque (20 ; 120) entre les côtés de la plaque pour guider la plaque lorsque la plaque se déplace dans ledit intervalle.

10. Dispositif (10) selon la revendication 9, dans lequel chacune des cames (30) comprend une tige montée sur le support (12) au niveau de la partie aval dudit intervalle.

11. Plaque (20 ; 120) pour une utilisation dans un dispositif (10) pour connecter et déconnecter de manière sélective des tubes en matière plastique (18 ; T), la plaque ayant la forme d'une plaque plate chauffée comprenant des cotés opposés (22, 22), chacun des cotés comprenant un godet s'étendant vers l'extérieur (28 ; 128), chacun des godets comprenant une extrémité ouverte et étant par ailleurs fermé de manière à former une poche de ramassage, et chacun des godets (28 ; 128) étant situé dans la même partie de son côté respectif (22) que l'autre des godets, d'où il résulte que les godets (28 ; 128) sont symétriques l'un par rapport à l'autre.

12. Plaque selon la revendication 11, comprenant une aile (26 ; 126) s'étendant vers l'extérieur à partir de chaque côté (22), séparée et sensiblement dans l'alignement de l'un respectif des godets (28 ; 128).

13. Plaque selon la revendication 12, dans laquelle les godets (28 ; 128) et les ailes (26 ; 126) sont situés à l'extrémité supérieure de chacun des côtés (22), une encoche (24 ; 124) étant formée dans l'extrémité inférieure de la plaque (20 ; 120).

14. Plaque selon l'une quelconque des revendications 11 à 13, comprenant une fente sensiblement rectiligne (50 ; 150) s'étendant à partir d'un bord de la plaque et traversant complètement la plaque, ladite fente étant sensiblement dans l'alignement et en aval des godets (28 ; 128).

15. Plaque selon l'une quelconque des revendications 11 à 14, comprenant une ouverture (136) traversant complètement la plaque, et un matériau de détection (138) recouvrant ladite ouverture (136).
